# EUROPEAN PATENT APPLICATION

(11) **EP 4 778 509 A1**
(43) Date of publication of application: **22.07.2026**
(21) Application number: 25193841.1
(22) Date of filing: 04.08.2025
(51) Int. Cl.: A61K 8/9794, A61K 9/10, A61P 17/02, A61P 17/16, A61P 17/18, A61K 9/19, A61K 8/14, A61K 8/97, A61K 36/898, A61Q 19/02, A61Q 19/08

(54) **ORCHID EXOSOME COMPOSITION FOR ENHANCING SKIN BARRIER, PROMOTING SKIN WHITENING AND ANTI-WRINKLE, METHOD THEREOF, FREEZE-DRIED CRYSTAL STRUCTURE, AND KIT STRUCTURE OF CARE PRODUCT**

(30) Priority: 20.01.2025 TW 114102279
(71) Applicant: Pegavision Corporation, Taoyuan City 333 (TW); FacialBeau International Corporation, New Taipei City 242 (TW)
(72) Inventor: PAN, I-Hong, 333 TAOYUAN CITY (TW); CHUANG, Kai-An, 333 TAOYUAN CITY (TW); YAO, Hsin-Jan, 333 TAOYUAN CITY (TW); TSAI, Pei-Yin, 333 TAOYUAN CITY (TW); LI, Kuei-Chang, 333 TAOYUAN CITY (TW)
(74) Representative: Winter, Brandl - Partnerschaft mbB

(57) **Abstract**

Provided is a composition for enhancing skin barrier, promoting skin whitening and anti-wrinkle, characterized in comprising exosomes isolated from an orchid as an active ingredient, wherein the orchid is Phalaenopsis aphrodite. Also provided is a freeze-dried crystal structure, including a main body composed of an excipient and the exosome as above mentioned. Also provided is a kit structure of care product including the freeze-dried crystal structure to achieve enhancing skin barrier, promoting skin whitening and anti-wrinkle.

## Description

### BACKGROUND

### Field of Invention

The present invention relates to exosome composition, method thereof, crystal structure, and kit structure. More particularly, the present invention relates to orchid exosome composition for enhancing skin barrier, promoting skin whitening and anti-wrinkle, method thereof, freeze-dried crystal structure, and kit structure of care product.

### Description of Related Art

Aging has long been a concern, increased fine wrinkles, decreased skin elasticity/atrophy, and accompanying itching are symptoms of skin aging. There are two types of causes of skin aging, intrinsic aging which occurs over time, and extrinsic aging which is caused by environmental factors (long-term exposure to sunlight and smoking). Changes in the activity of antioxidant enzymes are a physiological phenomenon in the early stages of aging. For example, hydrogen peroxide (H₂O₂) is a common reactive oxygen species (ROS) in the skin and is mainly removed by catalase. Studies have shown that in the dermis of older people, peroxidase activity is lower and hydrogen peroxide concentration increases. Therefore, when the skin's endogenous antioxidant capacity decreases with age, aging skin becomes more vulnerable to damage from external ultraviolet rays, pollution, and microorganisms, causing damage to the skin barrier and triggering inflammation inside the skin.

Since the epidermis is more exposed to external stimuli than the dermis, the keratinocytes in the epidermis have a higher burden of reactive oxygen species. These unstable free radical molecules attack lipids, proteins and DNA in skin cells, causing damage to the cell structure, weakening the skin's barrier function, and making the skin more vulnerable to damage from the external environment. Reactive oxygen species also promote the degradation of collagen and elastin, two proteins that are key components in maintaining skin firmness and elasticity. When they are damaged, the skin loses elasticity, becomes loose, and develops wrinkles and fine lines; in addition, free radicals can make the skin dull and rough, accelerating the aging process of the skin.

Therefore, how to strengthen the skin barrier function to maintain the stability of skin health, the related art really needs to be improved.

### SUMMARY

The present disclosure provides a composition for enhancing skin barrier, promoting skin whitening and anti-wrinkle, comprising exosomes isolated from an orchid as an active ingredient, wherein the orchid is *Phalaenopsis aphrodite.*

In some embodiments, the exosomes are isolated from a flower part and a peduncle of the orchid.

In some embodiments, an average particle size of the exosomes is from 20 nm to 500 nm.

In some embodiments, a concentration of the exosomes comprises from 1×10⁶ particles/mL to 1×10¹² particles/mL.

In some embodiments, a transmembrane protein of the exosomes comprises CD9.

In some embodiments, the composition is for use in skin regeneration, wrinkle reduction, anti-aging, skin moisturizing, skin elasticity improvement, wound repair, skin firming, skin whitening, or a combination thereof.

In some embodiments, the composition is for use in improving skin cell damage and collagen loss caused by oxidative stress, increasing expressions of genes comprising FBLN5, CERS3, COL13A1, COL15A1, IL-6, HAS1, CDSN, or a combination thereof in skin cells, increasing free radical scavenging ability, inhibiting melanin production, or a combination thereof.

The present disclosure also provides a method of enhancing skin barrier, promoting skin whitening, and anti-wrinkle, comprising administering to a subject in need thereof an effective amount of exosomes, wherein the exosomes are isolated from an orchid, wherein the orchid is *Phalaenopsis aphrodite.*

In some embodiments, the exosomes are prepared by the following steps: taking a flower part and a peduncle of the orchid; mixing water or a low salt buffer with the flower part and the peduncle of the orchid and then crushing to obtain a crude extract; filtering the crude extract to obtain a crude filtrate; and separating the crude filtrate by tangential flow filtration to obtain the exosomes.

The present disclosure also provides a freeze-dried crystal structure, comprising: a body consisting of an excipient; and exosomes isolated from an orchid and the orchid being *Phalaenopsis aphrodite,* wherein the exosomes uniformly disperse in the body.

In some embodiments, the excipient comprises trehalose, sodium hyaluronate, collagen, or a combination thereof.

In some embodiments, the body consists of the excipient and forms a reticular structure, and the exosomes uniformly disperse in the reticular structure.

In some embodiments, the freeze-dried crystal structure further comprises an active ingredient uniformly dispersing in the reticular structure.

In some embodiments, the active ingredient comprises peptides, growth factors, vitamin C, plant extracts, or a combination thereof.

In some embodiments, the exosomes are double-membrane structure.

In some embodiments, a transmembrane protein of the exosomes comprises CD9.

In some embodiments, an average particle size of one of the exosomes is from 20 nm to 500 nm.

In some embodiments, the freeze-dried crystal structure is prepared by the following steps: mixing the exosomes and the excipient to obtain a formula; and freeze-drying the formula to obtain the freeze-dried crystal structure.

The present disclosure also provides a kit structure of care product, comprising: a first container; a first accommodation space located in the first container; and the freeze-dried crystal structure as above mentioned located in the first accommodation space.

In some embodiments, the kit structure of care product further comprises a second container; a second accommodation space located in the second container; and a liquid excipient located in the second accommodation space, wherein the liquid excipient comprises water, saline solution, essence, or a combination thereof.

### BRIEF DESCRIPTION OF THE DRAWINGS

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion. The disclosure can be more fully understood by reading the following detailed description of the embodiment, with reference made to the accompanying drawings as follows:
Fig. 1 is a transmission electron microscopy image of an orchid exosome according to some embodiments of the present disclosure.
Fig. 2 is a bar graph showing the size and concentration of orchid exosomes according to some embodiments of the present disclosure.
Fig. 3 is a high performance liquid chromatography (HPLC) chromatogram of the orchid exosomes according to some embodiments of the present disclosure.
FIG. 4 is a bar graph showing a test of the protective activity of the orchid exosomes on keratinocytes according to some embodiments of the present disclosure. The experimental results were statistically compared among the groups using one-way ANOVA, **p<0.01, ****p<0.0001, and the results were presented as mean ± standard deviation (mean ± S.D.) (N=2).
Fig. 5 is a microscopic image of the orchid exosomes protecting keratinocytes according to some embodiments of the present disclosure. Cell images were recorded at a magnification of 10×10.
Fig. 6A is a photograph of the orchid exosomes inhibiting melanin production in melanocytes and a bar graph of melanin content in the supernatant according to some embodiments of the present disclosure.
Fig. 6B is a photograph of the orchid exosomes inhibiting melanin production in melanocytes and a bar graph of intracellular melanin content according to some embodiments of the present disclosure.
Fig. 7 is a heat map of gene expression among groups in a keratinocyte platform according to some embodiments of the present disclosure. The larger the value is, the more the gene is expressed, and the smaller the value is, the less the gene is expressed. The bar graph shows the comparison of the expression levels of genes related to skin physiology.
Fig. 8A is a bar graph showing the expression levels of FBLN5, CERS3, COL13A1, COL15A1 and IL6 genes among groups in the keratinocyte platform according to some embodiments of the present disclosure. FPKM is the abbreviation for Fragments Per Kilobase per Million.
Fig. 8B is a bar graph showing the expression levels of skin physiology-related genes HAS1 and CDSN in different groups under a fibroblast platform according to some embodiments of the present disclosure.
Fig. 9 is a bar graph showing the results of a skin irritation test of the orchid exosomes according to some embodiments of the present disclosure.
Fig. 10 is a bar graph showing the eye irritation test results of the orchid exosomes according to some embodiments of the present disclosure.
Fig. 11 is a schematic view of a freeze-dried crystal structure according to some embodiments of the present disclosure.
Fig. 12 is a schematic view of a kit structure of a care product according to some embodiments of the present disclosure.

### DETAILED DESCRIPTION

The following disclosure provides detailed description of many different embodiments, or examples, for implementing different features of the provided subject matter. These are, of course, merely examples and are not intended to limit the invention but to illustrate it. In addition, various embodiments disclosed below may combine or substitute one embodiment with another, and may have additional embodiments in addition to those described below in a beneficial way without further description or explanation. In the following description, many specific details are set forth to provide a more thorough understanding of the present disclosure. It will be apparent, however, to those skilled in the art, that the present disclosure may be practiced without these specific details.

Further, spatially relative terms, such as "beneath," "over" and the like, may be used herein for ease of description to describe one element or feature's relationship to another element(s) or feature(s) as illustrated in the figures. The spatially relative terms are intended to encompass different orientations of the device in use or operation in addition to the orientation depicted in the figures. The apparatus may be otherwise oriented (rotated 90 degrees or at other orientations) and the spatially relative descriptors used herein may likewise be interpreted accordingly.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising", or "includes" and/or "including" or "has" and/or "having" when used in this specification, specify the presence of stated features, regions, integers, steps, operations, elements, and/or components, but do not preclude the presence or addition of one or more other features, regions, integers, steps, operations, elements, components, and/or groups thereof.

Further, when a number or a range of numbers is described with "about," "approximate," and the like, the term is intended to encompass numbers that are within a reasonable range considering variations that inherently arise during manufacturing as understood by one of ordinary skill in the art. For example, the number or range of numbers encompasses a reasonable range including the number described, such as within +/-10% of the number described, based on known manufacturing tolerances associated with manufacturing a feature having a characteristic associated with the number. This repetition is for the purpose of simplicity and clarity and does not in itself dictate a relationship between the various embodiments and/or configurations discussed.

Exosomes are small and membrane-coated vesicles secreted by cells, ranging in size from about 20 nm to 500 nm. Exosomes are double-membrane structures composed of proteins, lipids and nucleic acids. Exosomes participate in intercellular messaging by transporting specific proteins, nucleic acids, and low molecular weight metabolites; because exosomes have relatively low immunogenicity, tumor tropism, and targeting of innate and acquired immune cells, exosomes have become the development trend of treatment and delivery systems for various diseases in recent years.

Theoretically, any cell can secrete exosomes. Exosomes from animal and human stem cells have been proven to have multiple functions such as promoting tissue regeneration, anti-aging, and immune regulation. Related research has also gradually expanded from medical functions to medical aesthetics, skin care products and other fields. However, there are no clear standards and specifications for animal stem cell exosomes at the regulatory level and skin care product application level.

Plants also secrete exosomes, called plant-derived exosome-like nanoparticles (PELNs), which are nanoscale vesicles secreted by plant cells, and contain substances such as DNA, small RNA (sRNA), microRNA (miRNA), and proteins. Since plant exosomes are of natural origin, can be mass-produced, have low immunogenicity, do not carry zoonotic pathogens, have higher bioavailability and potential biological activity, they are also a carrier tool for penetrating the skin. Active substances can enter the skin through skin-related glands (hair follicles, sebum, sweat glands), intercellular spaces and penetrating cells by exosomes. Thus, plant exosomes are considered to be an affordable source of production while being safer and more compliant than animal or human stem cell exosomes. Therefore, plant exosomes becoming opportunities for the development of innovative raw materials.

As used herein, "orchid exosomes" is also referred to as "*Phalaenopsis Aphrodite* exosome-like nanoparticles", or "*Phalaenopsis Aphrodite-*derived exosomes" in the disclosure, and refers to nano-sized vesicles secreted by cells into the extracellular space.

In some embodiments, the orchid exosomes are isolated from the orchid.

In some embodiments, the orchid exosomes have a particle size from 20 nm to 500 nm, for example, 25 nm, 30 nm, 35 nm, 40 nm, 45 nm, 50 nm, 60 nm, 70 nm, 80 nm, 90 nm, 100 nm, 150 nm, 200 nm, 250 nm, 300 nm, 350 nm, 400 nm, 450 nm, or any value between any two of these values.

In some embodiments, a concentration of the orchid exosomes comprises a range of from 1×10⁶ particles/mL to 1×10¹² particles/mL, for example, 3×10⁶ particles/mL, 5×10⁶ particles/mL, 7×10⁶ particles/mL, 9×10⁶ particles/mL, 1×10⁷ particles/mL, 3×10⁷ particles/mL, 5×10⁷ particles/mL, 7×10⁷ particles/mL, 9×10⁷ particles/mL, 1×10⁸ particles/mL, 3×10⁸ particles/mL, 5×10⁸ particles/mL, 7×10⁸ particles/mL, 9×10⁸ particles/mL, 1×10⁹ particles/mL, 5×10⁹ particles/mL, 1×10¹⁰ particles/mL, 5×10¹⁰ particles/mL, 1×10¹¹ particles/mL, 5×10¹¹ particles/mL, or any value between any two of these values.

In some embodiments, the orchid exosomes can be isolated by including, but not limited to ultracentrifugation, sucrose gradient centrifugation, microfiltration, tangential flow filtration, polymer precipitation, antibody magnetic bead separation, or a combination thereof.

Flowers are reproductive organs of angiosperms, and their biological function is to combine male sperm cells with female egg cells to produce seeds. A used herein, the term "flower part" at least includes petals, sepals, and columns. As used herein, the term "peduncle" is a stalk located below the flower part for supporting the inflorescence.

In some embodiments, the orchid exosomes are obtained by the following steps including: taking a flower part and a peduncle of the orchid; mixing water or a low salt buffer with the flower part and the peduncle of the orchid and then crushing to obtain a crude extract; filtering the crude extract to obtain a crude filtrate; and separating the crude filtrate by tangential flow filtration to obtain the orchid exosomes.

In some embodiments, the orchid exosomes is as an active ingredient for use in enhancing skin barrier and anti-wrinkle.

In some embodiments, the present disclosure provides a composition for use in enhancing skin barrier and anti-wrinkle.

In some embodiments, the composition is a cosmetic composition.

In some embodiments, the cosmetic composition may contain a functional additive and an ingredient commonly included in a cosmetic composition. The functional additive may include, but is not limited to, a polypeptide, a polysaccharide, a water-soluble vitamin, an oil-soluble vitamin, or a combination thereof. In addition, an oil, a fat, a humectant, an emollient, a surfactant, an organic or inorganic pigment, an organic powder, a UV absorbent, an antiseptic, a sterilizer, an antioxidant, a plant extract, a pH control agent, an alcohol, a colorant, a flavor, a blood circulation promoter, a cooling agent, an antiperspirant, purified water may be further contained.

In some embodiments, the composition is a pharmaceutical composition.

In some embodiments, the pharmaceutical composition includes the orchid exosomes as an active ingredient, administered to an individual either orally or parenterally. In some embodiments of the present disclosure, the orchid exosomes are formulated for administration to an individual in an oral dosage form selected from the group consisting of solutions, suspensions, emulsions, powders, lozenges, pills, syrups, lozenges, tablets, chewing gum, and capsules.

In some embodiments, the pharmaceutical composition includes the orchid exosomes as an active ingredient and pharmaceutical acceptable carriers including, but is not limited to water, alcohols, glycol, preserving agents, antioxidants, solvent, emulsifier, suspending agent, decomposer, binding agent, excipient, stabilizing agent, chelating agent, diluent, gelling agent, preservative, lubricant, absorption enhancers, active agents, humectants, odor absorbers, fragrances, pH adjusting agents, occlusive agents, emollients, thickeners, solubilizing agents, penetration enhancers, anti-irritants, colorants, propellants, surfactant, and other similar or applicable carriers for the present invention.

In some embodiments, the composition is a food composition.

In some embodiments, the food composition can be, for example, added to edible materials in the form of food additives to prepare a food product for human or animal consumption. Food composition includes, but is not limited to, general foods, health foods, beverages, nutritional supplements, dairy products or feeds, etc. In some examples of oral dosage forms, the food composition may optionally include food-acceptable carriers, excipients and/or additives. In other examples, the dosage form of the food composition may include, but is not limited to, powders, tablets, granules, suppositories, microcapsules, ampoules, liquid sprays or suppositories.

A number of examples are provided herein to elaborate the gel composition of the instant disclosure. However, the examples are for demonstration purpose alone, and the instant disclosure is not limited thereto.

The present disclosure provides a composition for use in enhancing skin barrier, and anti-wrinkle, comprising the orchid exosomes as active ingredient. The composition is for use in skin regeneration, wrinkle reduction, anti-aging, skin moisturizing, skin elasticity improvement, wound repair, or a combination thereof.

### Example

Although a series of operations or steps are used below to describe the method disclosed herein, an order of these operations or steps should not be construed as a limitation to the present invention. For example, some operations or steps may be performed in a different order and/or other steps may be performed at the same time. In addition, all shown operations, steps and/or features are not required to be executed to implement an embodiment of the present invention. In addition, each operation or step described herein may include a plurality of sub-steps or actions.

For the sake of clarity, features and elements that are well known in the art and are not necessary for an understanding of the principles described have been omitted.

### Preparation Example 1 Method for preparing orchid exosomes

Flower part and peduncle of the orchid was provided, and 1.5 folds weight of water was added and crushed with a blender to obtain a crude extract. The crude extract was separated into solid and liquid, and the liquid part was filtered through a 0.22 µm filter membrane to remove impurities and obtain the crude filtrate. The crude filtrate was concentrated and separated into orchid exosomes or orchid-derived exosomes (OR-TFF) by tangential flow filtration (TFF) with a 100 kDa filter membrane. The appearance of the purified orchid exosomes was confirmed by electron microscope, and the particle size and concentration of the orchid exosomes were analyzed by a nanoparticle size analyzer.

### Example 1: Characterization of the orchid exosomes

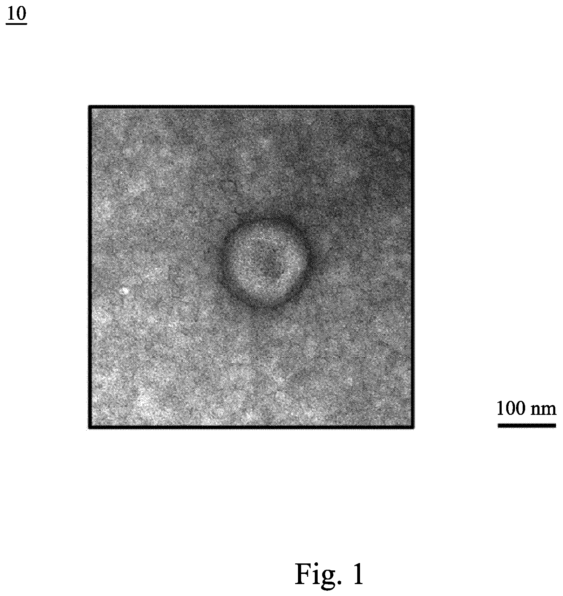

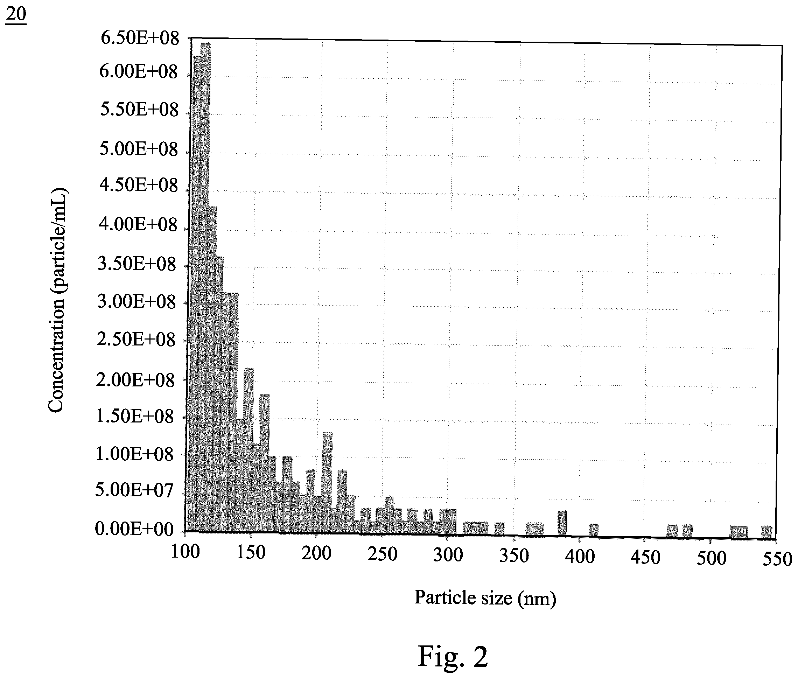

The size and properties of the orchid exosomes obtained in Preparation Example 1 were analyzed by transmission electron microscope (TEM). Fig. 1 is a transmission electron microscopy image 10 of an orchid exosome according to some embodiments of the present disclosure, and Fig. 1 shows that orchid exosomes had diameters ranging from 20 nm to 500 nm and were roughly spherical. Fig. 2 is a bar graph 20 showing the size and concentration of orchid exosomes according to some embodiments of the present disclosure, and the concentration of orchid exosomes per unit volume of 1 milliliter (mL) was confirmed by nanoparticle tracking analysis (NTA).

### Example 2: Analysis of specific marker CD9 transmembrane protein of the orchid exosomes

Previous studies have reported that plants contain transmembrane structures similar to animal exosome CD9, so this example was attempted to use a CD9 detection kit (ExoELISA-ULTRA Complete Kit, Cat No. EXEL-ULTRA-CD9-1, System Bioscience, UK) for marker detection. The results were shown in Table 1 below, compared with the color values of the exosome standard, the orchid exosomes obtained in Preparation Example 1 can also be seen to have a color reaction under this platform. Therefore, the orchid exosomes obtained in Preparation Example 1 not only has a particle size and image that comply with the characteristics of exosomes, but also has a lipid bilayer membrane that contains the common exosome-specific marker CD9 transmembrane protein.

**Table 1**

| Test sample (particles/mL) | Value of color reaction (OD₄₅₀) |
|---|---|
| standard sample | |
| 5.42×10¹⁰ | 1.44±0.06 |
| 3.61×10¹⁰ | 1.43±0.08 |
| 2.71×10¹⁰ | 1.34±0.09 |
| 1.81×10¹⁰ | 1.18±0.03 |
| 0.90×10¹⁰ | 0.70±0.02 |
| 0.45×10¹⁰ | 0.33±0.03 |
| 0.23×10¹⁰ | 0.16±0.01 |
| 0 | 0.06±0.02 |

| orchid exosomes | |
|---|---|
| 2.94×10¹⁰ | 0.21±0.01 |

### Example 3: HPLC analysis of secondary metabolites of orchid exosomes

The main ingredients of the plant derived exosome are protein, lipid, nuclei acid and specific secondary metabolites of different plants. The secondary metabolites derived from plant have specific bioactive materials. Compared with the ordinary plant extract, plant exosomes have unique phospholipid bilayer structure to effetely preserve the active ingredient, to protect the secondary metabolites from being easily affected or damaged by external environment, and to enhance bioavailability. Therefore, in order to understand the characteristics of the secondary metabolites in the orchid exosomes, HPLC was selected to analyze the components of the secondary metabolites in exosomes of *Phalaenopsis aphrodite.*

Sample preparation: The orchid exosomes was prepared in Preparation Example 1 and filtered through a 0.45 µm filter membrane to perform HPLC analysis.
Mobile phase A: 0.1% phosphoric acid/water
Mobile phase B: acetonitrile

**Table 2**

| Time (min) | Solution A (%) | Solution B (%) |
|---|---|---|
| 0 | 90 | 10 |
| 5 | 90 | 10 |
| 15 | 80 | 20 |
| 25 | 0 | 100 |
| 26 | 90 | 100 |
| 35 | 90 | 10 |

Chromatographic column: Shim-pack VP-ODS C18 column (2.0*150 mm, 5.0 µm, Shimadzu Corporation, Kyoto, japan)
Flow rate: 0.3 mL/min, UV=254 nm
Column Temp: 40°C, Injection Volume: 10 µL

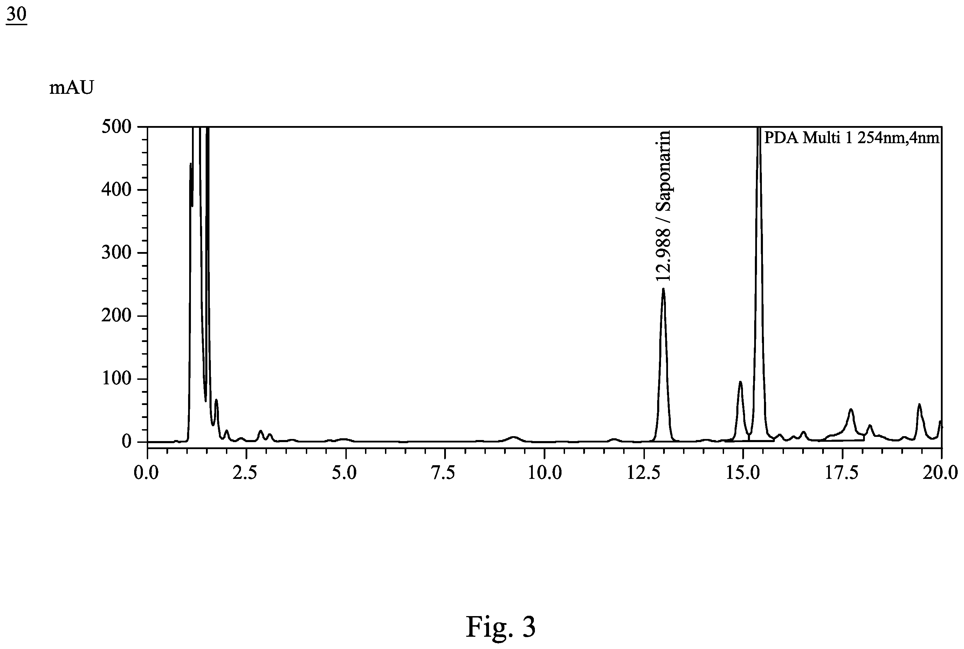

Fig. 3 is a HPLC chromatogram 30 of the orchid exosomes according to some embodiments of the present disclosure. The results showed that at a concentration of 2.38×10¹⁰ in Preparation Example 1, orchid exosomes had a peak with the same retention time as the saponin (IUPAC name: 5-Hydroxy-6-(β-D-glucopyranosyl)-7-(β-D-glucopyranosyloxy)flavone) standard, indicating that the orchid exosomes contain polyphenol components of saponin.

### Example 4: Protective activity of skin keratinocytes

In this example, hydrogen peroxide was used to simulate oxidative stress to induce damage to human HaCaT keratinocytes, and the protective activity on the cells was evaluated after the orchid exosomes obtained in Preparation Example 1 was added. HaCaT keratinocytes were cultured in DMEM containing 100 units/mL penicillin, 100 µg/mL streptomycin, and 10% fetal bovine serum in an environment of 5% CO₂ and 37°C. First, 1×10⁴ cells were cultured in a 96-well culture plate for 24 hours. The supernatant was removed on the second day, and 2.5% (i.e., 5.95×10⁸ particles/mL), 5% (i.e., 1.19×10⁹ particles/mL), and 10% (i.e., 2.38×10⁹ particles/mL) of the orchid exosomes from Preparation Example 1 were added to the cell culture medium for 1 hour. Then, hydrogen peroxide was added to make the final concentration 150 µM and reacted in an incubator for 16 hours. On the third day, the cells were reacted with 0.5 mg/mL 3-(4,5-dimethylthiazol-2-yl)-2,5-diphenyltetrazolium bromide (MTT) for 1 hour and then dissolved with dimethyl sulfoxide (DMSO). The cell survival rate of each group was shown in percentage of OD₅₇₀ absorbance compared with that of the control group.

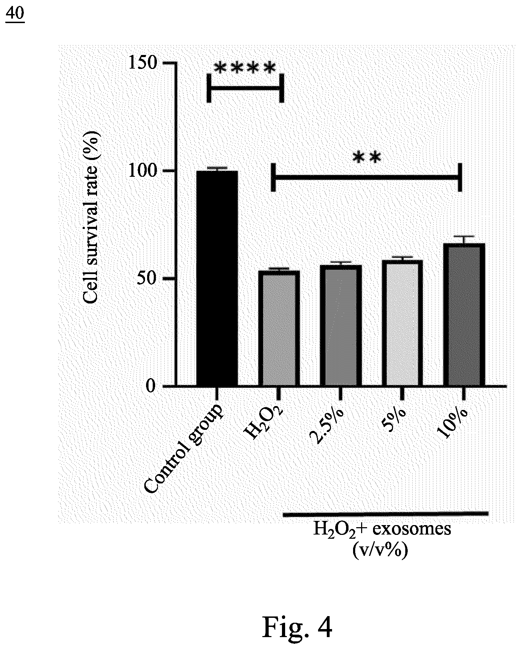

FIG. 4 is a bar graph 40 showing a test of the protective activity of the orchid exosomes on keratinocytes according to some embodiments of the present disclosure. The results showed that compared with the control group, hydrogen peroxide caused a decrease in cell survival rate (53.6±1.1%). After adding 2.5%, 5% and 10% of the orchid exosomes obtained from Preparation Example 1, the survival rate recovered to 56.4±1.4%, 58.8±1.1%, and 66.4±3.1%, respectively (p<0.01), indicating that the orchid exosomes had the activity of protecting cells and retaining collagen under the platform of HaCaT damage induced by oxidative stress.

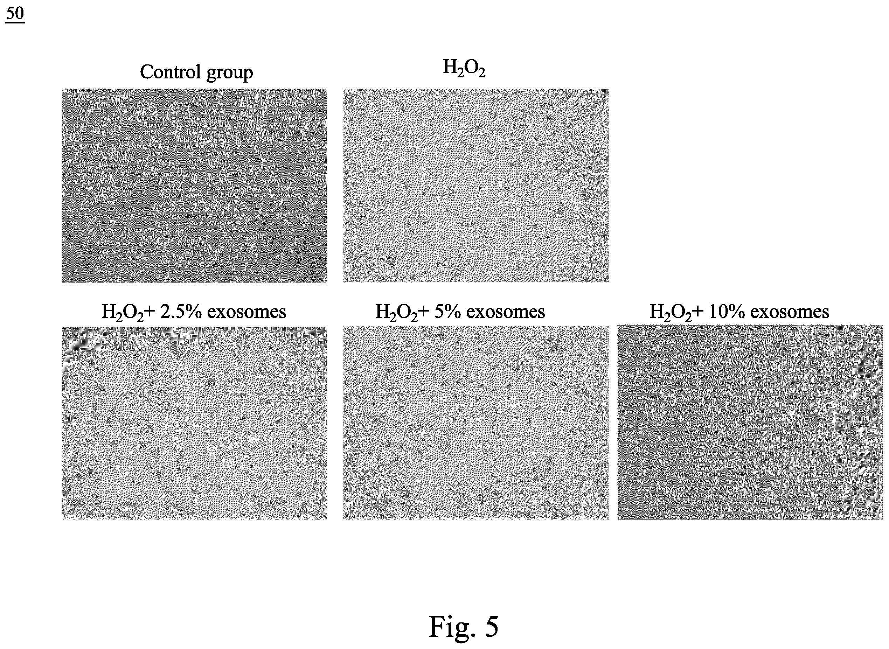

In addition, after pre-treatment with different concentrations of the orchid exosomes obtained from Preparation Example 1, hydrogen peroxide was added to induce cell damage, and Sirius Red staining was performed 16 hours later, and cell images were observed under a microscope. Fig. 5 is a microscopic image 50 of the orchid exosomes protecting keratinocytes according to some embodiments of the present disclosure. The results showed that compared with the control group (Control), the number of HaCaT cells and collagen were significantly reduced under the induction of oxidative stress (hydrogen peroxide). However, after the orchid exosomes of Preparation Example 1 were administered, the number of cells and the collagen content had a tendency to recover with the increase of the concentration of the orchid exosomes. Therefore, it was confirmed that the orchid exosomes had the effects of skin regeneration and wrinkle reduction.

### Example 5: Melanin inhibitory activity

In this example, 1-methyl-3-isobutylxanthine (IBMX) was used to induce mouse B16-F0 melanocytes to produce melanin, and the inhibitory activity of the melanin production was evaluated after the orchid exosomes obtained from Preparation Example 1 were added. B16-FO melanocytes were cultured in DMEM containing 100 units/mL penicillin, 100 µg/mL streptomycin, 72 µg/mL tyrosine and 10% fetal bovine serum in a 5% CO₂, 37°C environment. First, 1×10⁴ cells were cultured in a 35 mm culture dish for 24 hours. On the second day, after removing the supernatant, 2.5% (i.e., 5.95×10⁸ particles/mL), 5% (i.e., 1.19×10⁹ particles/mL), or 10% (i.e., 2.38×10⁹ particles/mL) of the orchid exosomes from Preparation Example 1 with IBMX (100 µM) were added to the cell culture medium for co-reaction for 3 days. After co reaction for 3 days, the cell culture supernatant and cell pellet were photographed and recorded. To quantify the content of melanin, each group of cells was harvested with 0.25% Trypsin-EDTA, washed twice with 1×PBS, and lysed with 1N NaOH and reacted at 80°C for 30 minutes. Absorbance readings of the cell lysate and supernatant were taken by OD₄₀₅.

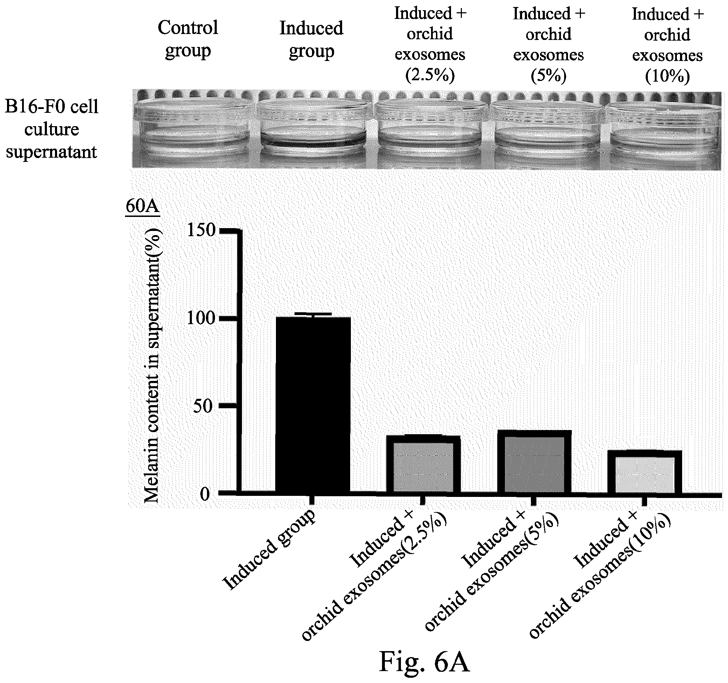

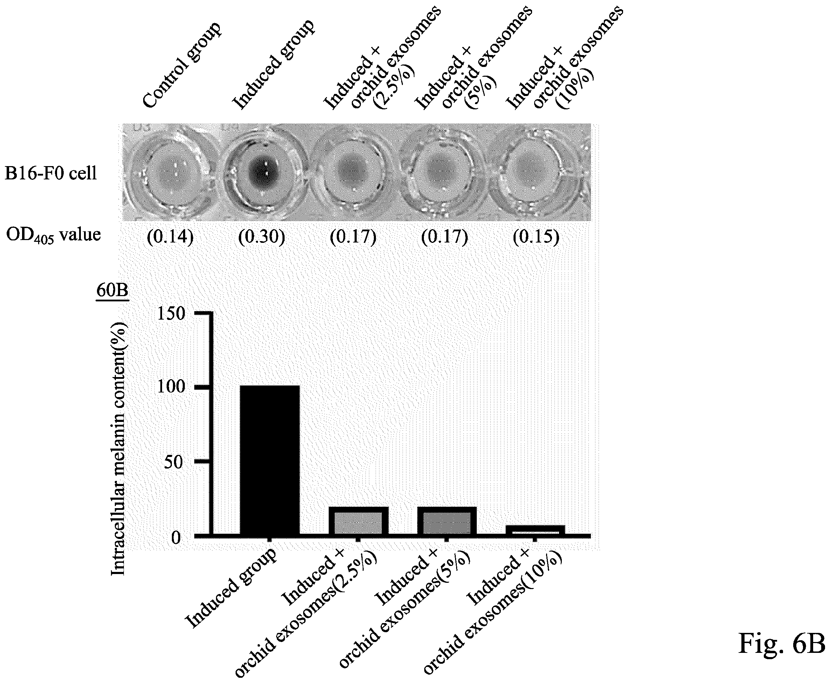

Fig. 6A is a photograph 60A of the orchid exosomes inhibiting melanin production in melanocytes and a bar graph of melanin content in the supernatant according to some embodiments of the present disclosure. The results showed that in the supernatant, compared with the control group, IBMX caused the cells to produce and secrete melanin into the culture medium. After adding 2.5%, 5% and 10% of the orchid exosomes obtained in Preparation Example 1, the percentage of melanin content (with the induced group being 100±3.1%) were 32.6±1.1%, 36.1±0.2% and 24.9±0.5% left, respectively. Fig. 6B is a photograph 60B of the orchid exosomes inhibiting melanin production in melanocytes and a bar graph of intracellular melanin content according to some embodiments of the present disclosure. In the cell, compared with the control group, IBMX induced the cells to produce melanin (the induced group calculated as 100%). After adding 2.5%, 5% and 10% of the orchid exosome obtained in Preparation Example 1, the percentage of melanin were 18.8%, 18.8% and 6.3% left, respectively, indicating that the orchid exosomes can inhibit the production of melanin in B16-FO cells induced by IBMX.

### Example 6: Effects on the expression of anti-aging/moisturizing/repair genes in skin keratinocytes

In this example, the same method as Example 4 was used to pre-treat HaCaT keratinocytes with the 10% orchid exosomes from Preparation Example 1, and then hydrogen peroxide was added to induce cell damage reaction for 16 hours; or HS-68 fibroblasts were treated with the orchid exosomes for 24 hours and then RNA was extracted. After nucleic acid quality testing and library construction, nucleic acid sequencing was performed using the NovaSeq X Plus Sequencing System to observe the effect of the orchid exosomes on gene expression under this platform.

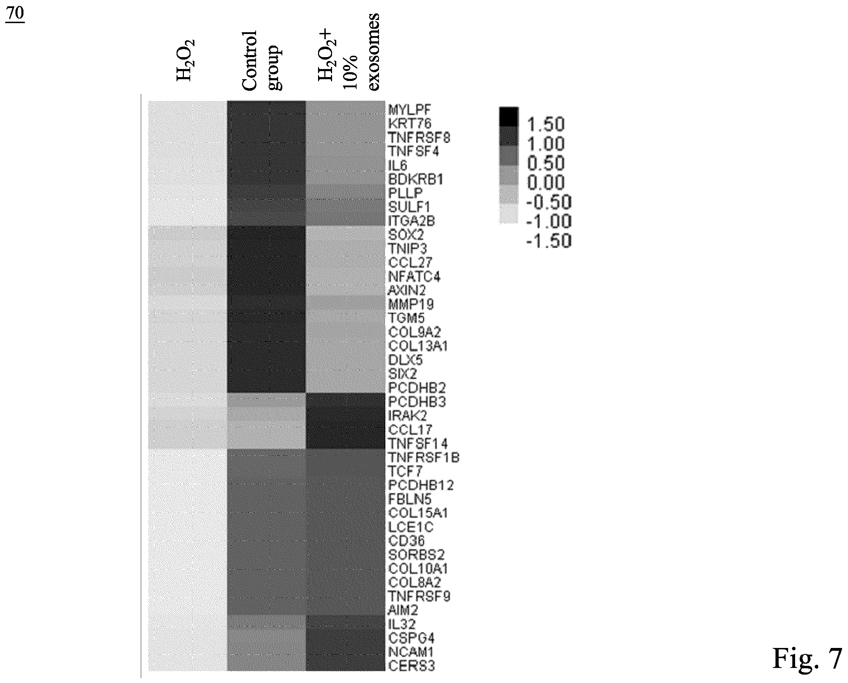

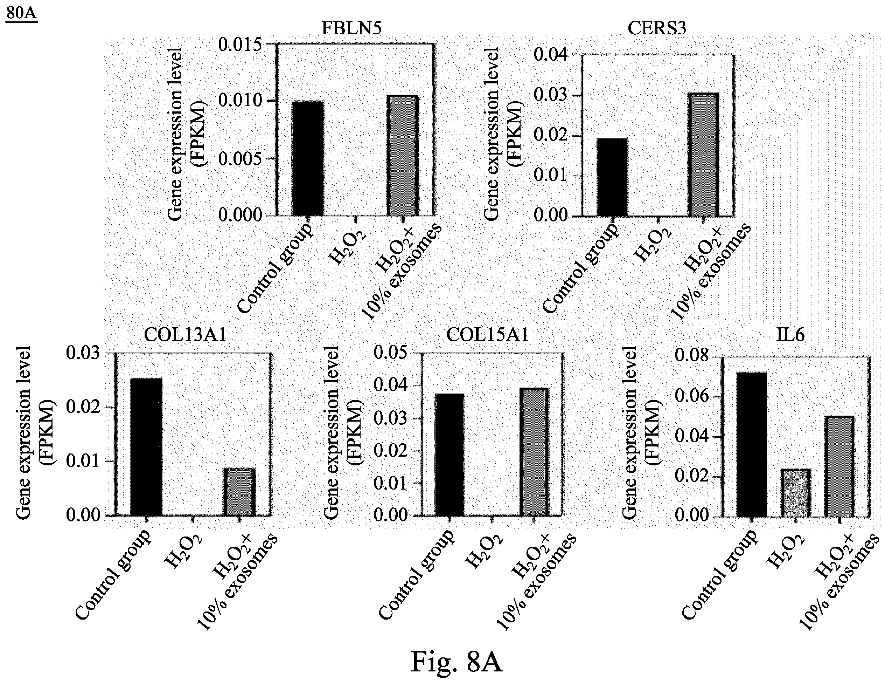

Fig. 7 is a heat map 70 of gene expression among groups in a keratinocyte platform according to some embodiments of the present disclosure. The result is a heat map of gene expression related to skin cell physiology (GO:0030216, GO:0008544, GO:0042438, GO:0006955, GO:0006954, GO:0070098, GO:0030198, GO:0030574, GO:0007155, GO:0009611, GO:0071711, GO:0061436, GO:0006583, GO:0042438, GO:0008283, GO:0040037) classified by the Gene Ontology database. Fig. 8A is a bar graph 80A showing the expression levels of FBLN5, CERS3, COL13A1, COL15A1 and IL6 genes among groups in the keratinocyte platform according to some embodiments of the present disclosure. Compared with the hydrogen peroxide group, a total of 41 genes showed recovery results under the administration of the 10% orchid exosomes, among which: FBLN5 was related to anti-aging, CERS3 was a ceramide synthase gene related to moisturizing function, COL13A1 and COL15A1 were both important genes of the epidermal basement membrane and related to skin elasticity, and IL6 was related to skin wound repair.

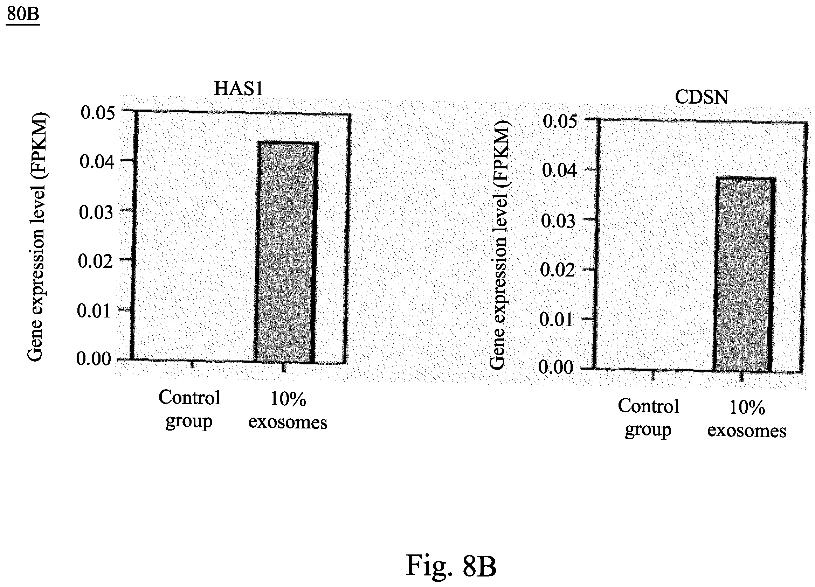

Fig. 8B is a bar graph 80B showing the expression levels of skin physiology-related genes HAS1 and CDSN in different groups under a fibroblast platform according to some embodiments of the present disclosure. In fibroblasts, the orchid exosomes were found to promote the HAS1 hyaluronic acid synthesis enzyme gene related to water retention, and the CDSN corneous desmosome gene (related to the connection between cells) related to skin firming.

The composition disclosed herein is effective in resisting skin damage and collagen breakage caused by environmental oxidative stress, and is for use in skin regeneration, skin elasticity improvement or wrinkle reduction. The composition also has the ability to increase the expression of FBLN5, CERS3, COL13A1, COL15A1 and IL-6 genes in skin cells, and has skin moisturizing ability, prevents skin aging, and performs wound repair.

### Example 7: Free radical scavenging ability assay

In this example, the free radical scavenging capacity assay kit (SunLong^{®} Biotech Co., LTD) was used to evaluate the free radical scavenging capacity. The experiment was conducted in a 96-well plate. 10 µL of the 100 v/v% orchid exosomes stock solution or its dilution from Preparation Example 1 was uniformly mixed with 190 µL of the prepared DPPH working solution. After 10 minutes of reaction, the OD₅₁₅ absorbance was detected by a Synergy H1 Hybrid Multi-Mode Microplate (Agilent BioTek^{®}). Finally, the scavenging efficiency of DPPH free radicals was calculated by the following formula: DPPH free radical scavenging rate DVC%=[[(A_{B} - (A_{T}-A_{C} )] ÷A_{B} ] ×100%.

**Table 3, Results of the DPPH free radical scavenging ability test of the orchid exosomes**

| Concentration | DPPH free radical scavenging efficiency (%) |
|---|---|
| orchid exosome (v/v) | |
| 12.5% | 3.5 ± 5.1% |
| 25% | 9.9 ± 6.4% |
| 50% | 13.6 ± 1.0% |
| 100% | 35.3 ± 3.9% |

| Vitamin C (µg/mL) | |
|---|---|
| 15.6 | 5.3 ± 2.0% |
| 31.25 | 13.7 ± 3.4% |
| 62.5 | 35.6 ± 8.1% |
| 125 | 60.0 ± 2.7% |
| 250 | 90.0 ± 0.3% |

### Example 8: Safety assessment

In this embodiment, a safety assessment test was conducted on an *in vitro* bionic skin platform, and a skin irritation test was conducted on the orchid exosomes of Preparation Example 1. The skin irritation test included the following operations. This test was conducted on a human skin model using the test kit (EpiSkin^{™} KIT, WPISKIN/S/13) approved in TG 439 of the Organization for Economic Cooperation and Development (OECD). During the test, the orchid exosomes of Preparation Example 1 was dissolved in 1×PBS to a final concentration of 10 v/v%, and then directly added to the surface of the skin model. After 15 minutes of reaction, it was washed off, and the skin model was cultured for 42 hours. Finally, the effect of Preparation Example 1 or the positive control group (5wt% SDS) on cell activity was evaluated by cell viability analysis (MTT assay). The irritation evaluation standard was based on the United Nations Globally Harmonized System of Classification and Labeling of Chemicals (UN GHS) Type 2, and when the cell viability was ≤ 50%, it was considered to be skin irritating.

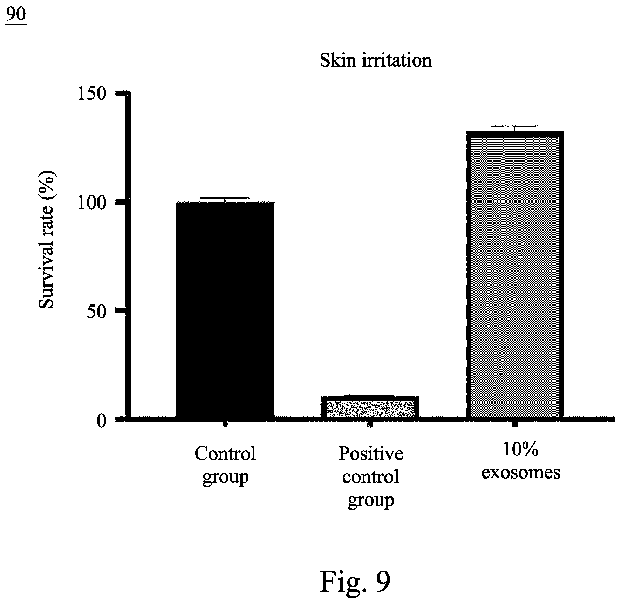

Fig. 9 is a bar graph 90 showing the results of a skin irritation test of the orchid exosomes according to some embodiments of the present disclosure, and Fig. 9 showed the skin irritation test results of the 10% orchid exosomes, control group and positive control group disclosed in the present disclosure. The experimental data were expressed as mean ± S.D., and the number of samples was 3. After the skin irritation test, the survival rate of the control group was 100.0±1.9%, the survival rate of the 10% orchid exosomes was 132.2±2.6%, and the positive control group was 10.5±0.2%. As shown in Fig. 9, the 10% orchid exosome of Preparation Example 1 was non-irritating in the skin irritation test.

Furthermore, this example also conducted a safety assessment test for eye irritation, and performed an eye irritation test on the orchid exosomes of Preparation Example 1. This test was conducted in accordance with the OECD TG 492 specification and a detection kit (LabCyte^{™} CORNEA-MODEL, Cat. No. 411324) was used for testing on a human corneal epithelial tissue model. During the test, the orchid exosomes of Preparation Example 1 was dissolved in 1×PBS to a final concentration of 10 v/v%, and then directly added to the surface of the corneal epithelial tissue model. After 60 minutes of reaction, it was washed off, and then the corneal epithelial tissue model was cultured for 24 hours. Finally, the effect of 10% orchid exosomes or the positive control group (ethanol) on cell activity was evaluated by cell viability analysis (WST-8 assay kit). The irritation evaluation standard was based on UN GHS type 2, and when the cell viability was ≤ 40%, it was considered to be eye irritating.

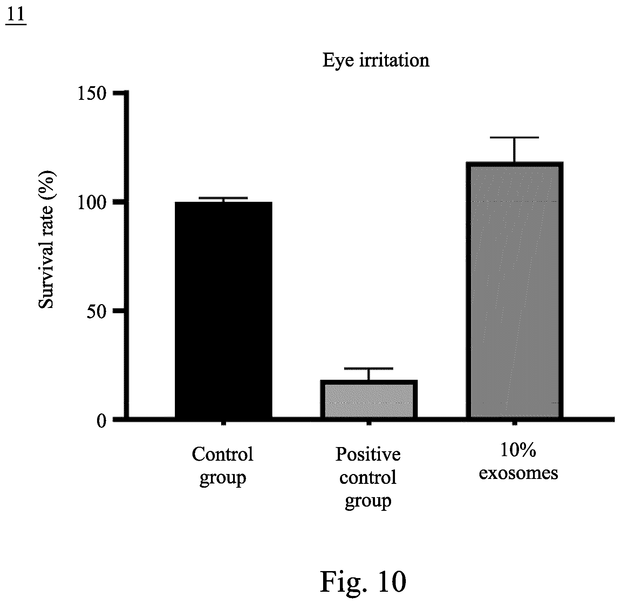

Fig. 10 is a bar graph 11 showing the eye irritation test results of the orchid exosomes according to some embodiments of the present disclosure, and Fig. 10 was the corneal epithelial tissue irritation test results of the 10% orchid exosomes, the control group and the positive control group disclosed in the present disclosure. The experimental data were represented by mean ± standard deviation (mean ± SD), and the number of samples was 3. After the eye irritation test, as shown in Fig. 10, the survival rate of the control group was 100.0 ± 1.8%, the survival rate of the 10% exosome was 118.4 ± 11.3%, and the positive control group was 18.2 ± 5.1%. As shown in Fig. 10, the 10% orchid exosomes of Preparation Example 1 was non-irritating in the eye irritation test.

### Example 9: Preparation of freeze-dried crystal structure of the orchid exosomes

The orchid exosomes (OR TFF) of Preparation Example 1 was used as a raw material and freeze-dried to prepare a freeze-dried crystal structure, so as to prevent the external environment and other factors from affecting the activity of the orchid exosomes, thereby improving the storage stability of the orchid exosomes and increasing the shelf life of the nano-sized orchid exosomes. Furthermore, the freeze-dried crystal structure can be quickly dissolved and mixed with essence or lotion for skin care.

The preparation method was as follows: the 10 v/v% orchid exosomes of Preparation Example 1, an excipient containing trehalose, sodium hyaluronate, collagen, and water were stirred until completely uniform to obtain a formula, which was first placed at -20°C for freezing. Next, the formula was placed in a freeze dryer for freeze drying to obtain a freeze-dried crystal structure. In some embodiments, the formula appeared as frozen crystal powder under macroscopic observation and had a plurality of crystal grain structures under microscopic observation. The freeze-dried crystal structure can be quickly dissolved and mixed with essence or lotion for skin care.

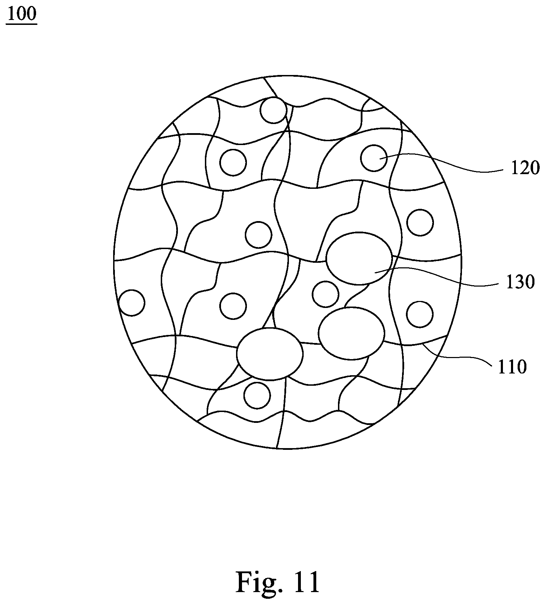

Fig. 11 is a schematic view of a freeze-dried crystal structure according to some embodiments of the present disclosure. Freeze-dried crystal structure 100 includes a body 110, exosomes 120, and an active ingredient 130. The body 110 consists of excipient including trehalose, sodium hyaluronate, collagen, or a combination thereof. In some examples, the body 110 consists of the excipient to form a reticular structure.

The exosomes 120 are derived from the orchid, on which the orchid is *Phalaenopsis aphrodite*; specificity, the exosomes 120 are obtained from the orchid exosomes form Preparation Example 1. The exosomes 120 uniformly disperse in the body 110. In some examples, exosomes 120 uniformly disperse in the body 110 to form in the reticular structure. In some examples, exosomes 120 are double-membrane structure, transmembrane protein of the exosomes 120 includes CD9, and an average particle size of the exosomes 120 is from 20 nm to 500 nm.

The active ingredient 130 uniformly disperse in the reticular structure formed by the body 110, and the active ingredient 130 includes peptides, growth factors, vitamin C, plant extracts, or a combination thereof.

### Example 10: Kit structure of care product

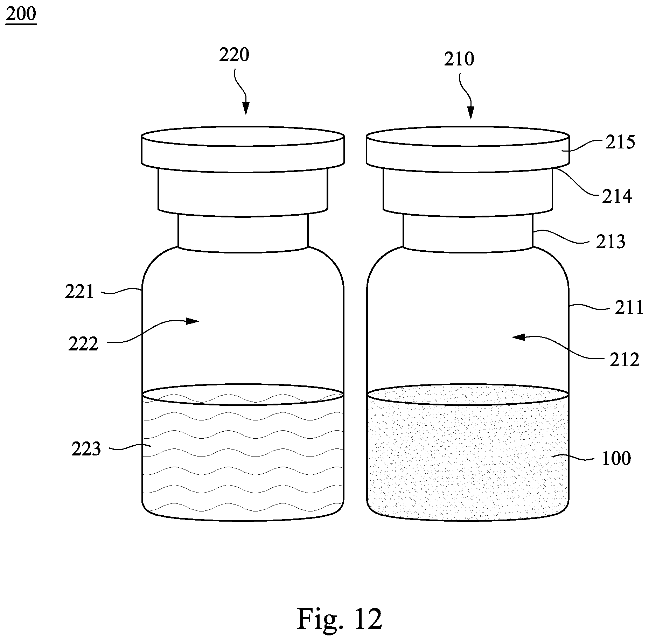

Fig. 12 is a schematic view of a kit structure of a care product according to some embodiments of the present disclosure. The kit structure of care product 200 includes a first container 210 and a second container 220. The first container 210 includes a bottle body 211, a first accommodation space 212, a bottleneck 213, a bottle mouth 214, and a bottle cap 215. Specificity, the bottle body 211 surrounds and forms the first accommodation space 212, so that the freeze-dried crystal structure 100 is accommodated in the first accommodation space 212. The bottleneck 213 is disposed on a top of the bottle body 211, and the bottle mouth 214 is formed at a top portion of the bottleneck 213. The bottle cap 215 is disposed at the bottle mouth 214 to seal the first container 210.

The second container 220 has a structure similar to the first container 210, thus the details will not be repeated. The second container 220 mainly includes the bottle body 221and the second accommodation space 222 are surrounded and formed by the bottle body 221. The liquid excipient 223 is located in the second accommodation space 222, in which the liquid excipient 223 includes water, saline solution, essence, or a combination thereof.

In some examples, the first container 210 and the second container 220 are ampoule, and respectively accommodate the freeze-dried crystal structure 100 and the liquid excipient 223. The liquid excipient 223 in the second container 220 was added into the first container 210 containing the freeze-dried crystal structure 100 before use. After the freeze-dried crystal structure 100 and the liquid excipient 223 were uniformly mixed, they can be directly applied to the skin.

The present disclosure provides a composition for enhancing skin barrier, promoting skin whitening, and anti-wrinkle, comprising the orchid exosomes as an active ingredient. The composition is for use in skin regeneration, wrinkle reduction, anti-aging, skin moisturizing, skin elasticity improvement, wound repair, or a combination thereof.

The present disclosure also provides a freeze-dried crystal structure for protecting the active ingredient, and the orchid exosomes are sealed by porous micro-gaps formed inside and on the surface of the freeze-dried crystal structure, so as to improve the storage stability of the active ingredient and extend the shelf life.

## Claims

1. A composition for enhancing skin barrier, promoting skin whitening and anti-wrinkle, **characterized in** comprising exosomes isolated from an orchid as an active ingredient, wherein the orchid is *Phalaenopsis aphrodite.*

2. The composition as claimed in claim 1, wherein the exosomes are isolated from a flower part and a peduncle of the orchid.

3. The composition as claimed in claims 1 or 2, wherein an average particle size of the exosomes is from 20 nm to 500 nm.

4. The composition as claimed in any one of claims 1 to 3, wherein a concentration of the exosomes comprises from 1×10⁶ particles/mL to 1×10¹² particles/mL.

5. The composition as claimed in any one of claims 1 to 4, wherein a transmembrane protein of the exosomes comprises CD9.

6. The composition as claimed in any one of claims 1 to 5, wherein the composition is for use in skin regeneration, wrinkle reduction, anti-aging, skin moisturizing, skin elasticity improvement, wound repair, skin firming, skin whitening, or a combination thereof.

7. The composition as claimed in any one of claims 1 to 6, wherein the composition is for use in improving skin cell damage and collagen loss caused by oxidative stress, increasing expressions of genes comprising FBLN5, CERS3, COL13A1, COL15A1, IL-6, HAS1, CDSN, or a combination thereof in skin cells, increasing free radical scavenging ability, inhibiting melanin production, or a combination thereof.

8. The composition as claimed in any one of claims 1 to 7, wherein the exosomes are prepared by the following steps:
taking a flower part and a peduncle of the orchid;
mixing water or a low salt buffer with the flower part and the peduncle of the orchid and then crushing to obtain a crude extract;
filtering the crude extract to obtain a crude filtrate; and
separating the crude filtrate by tangential flow filtration to obtain the exosomes.

9. A freeze-dried crystal structure (100), **characterized in** comprising:
a body (110) consisting of an excipient; and
exosomes (120) isolated from an orchid and the orchid being *Phalaenopsis aphrodite,* wherein the exosomes (120) uniformly disperse in the body.

10. The freeze-dried crystal structure (100) of claim 9, wherein the excipient comprises trehalose, sodium hyaluronate, collagen, or a combination thereof.

11. The freeze-dried crystal structure (100) of claims 9 or 10, wherein the body (110) consists of the excipient and forms a reticular structure, and the exosomes (120) uniformly disperse in the reticular structure.

12. The freeze-dried crystal structure of claim 11, further comprising an active ingredient uniformly dispersing in the reticular structure.

13. The freeze-dried crystal structure (100) of claim 12, wherein the active ingredient (130) comprises peptides, growth factors, vitamin C, plant extracts, or a combination thereof.

14. The freeze-dried crystal structure (100) of any one of claims 9 to 13, wherein an average particle size of one of the exosomes (120) is from 20 nm to 500 nm.

15. A kit structure (200) of care product, **characterized in** comprising:
a first container (210);
a first accommodation space (212) located in the first container (210); and
the freeze-dried crystal structure (100) as claimed in any one of claims 9 to 14 located in the first accommodation space (210).
